# EUROPEAN PATENT APPLICATION

(11) **EP 3 569 074 A1**
(43) Date of publication of application: **20.11.2019**
(21) Application number: 17892909.7
(22) Date of filing: 31.07.2017
(51) Int. Cl.: A24F 47/00

(54) **CIGARETTE CARTRIDGE, ATOMIZER AND ELECTRONIC CIGARETTE THEREOF**

(30) Priority: 19.01.2017 CN 201710037941
(71) Applicant: Changzhou Patent Electronic Technology Co., Ltd, Jiangsu 213001 (CN)
(72) Inventor: QIU, Weihua, Jiangsu 213125 (CN)
(74) Representative: Zaboliene, Reda
(86) International application number: PCT/CN2017/095286
(87) International publication number: WO 2018/133369

(57) **Abstract**

A cigarette cartridge, an atomizer and an electronic cigarette are provided. The cigarette cartridge includes a liquid storage tube, an elastic member and a first magnetic member. A liquid storage chamber is provided in the liquid storage tube. The cigarette cartridge is provided with a liquid guiding hole in communication with the liquid storage chamber. One end of the elastic member is fixed relative to the liquid storage tube, the other end of the elastic member elastically resists the first magnetic member, such that the first magnetic member is capable of moving under an action of the elastic member to cause the liquid guiding hole to be closed.

## Description

### TECHNICAL FIELD

The present application relates to the technical field of smoking simulation, and more particularly, to a cigarette cartridge, an atomizer and an electronic cigarette.

### BACKGROUND

In the cigarette cartridge of conventional electronic cigarettes, the liquid storage chamber of the cigarette cartridge generally has an opened structure. Therefore, when the cigarette cartridge is disassembled, the cigarette liquid in the liquid storage chamber will leak out, not only a pollution is caused, but also the user experience is influenced. Furthermore, when children disassemble the cigarette cartridge casually, an ingestion of the cigarette liquid by accident occurs easily, a potential safety hazard exists.

### SUMMARY

Accordingly, it is necessary to provide a cigarette cartridge, so that the cigarette liquid cannot leak out when the cigarette cartridge is separately placed. Further, an atomizer having the cigarette cartridge and an electronic cigarette having the atomizer are also provided.

The technical solution provided in the present application to solve the problem is as follows.

A cigarette cartridge includes a liquid storage tube, a liquid outlet device, an elastic member and a first magnetic member. The elastic member and the first magnetic member are received in the liquid outlet device. A liquid storage chamber is provided in the liquid storage tube. The liquid outlet device is provided with a liquid guiding hole in communication with the liquid storage chamber. The cigarette cartridge is further provided with a liquid outlet hole in communication with the liquid guiding hole. The first magnetic member is slidably received in the liquid outlet device. One end of the elastic member elastically resists an inner wall of the liquid outlet device, and the other end of the elastic member elastically resists the first magnetic member. The first magnetic member is capable of closing the liquid guiding hole under an action of the elastic member.

Further, the liquid outlet device is received in the liquid storage tube. The first magnetic member is slidable along an axial direction of the liquid storage tube.

Further, the liquid guiding hole is defined in a sidewall of the liquid outlet device. The liquid outlet hole is defined at a lower end of the liquid outlet device.

Further, a lower end of the liquid storage tube defines a though hole in communication with the liquid storage chamber, and a lower end of the liquid outlet device fixedly extends into the through hole of the liquid storage tube.

Further, the liquid outlet device is provided outside the liquid storage tube and connected to a lower end of the liquid storage tube. The first magnetic member is slidable along a radial direction of the liquid storage tube.

Further, the liquid guiding hole is defined in a connecting surface between the liquid outlet device and the liquid storage tube. The liquid outlet hole is defined in the liquid outlet device corresponding to the liquid guiding hole.

Further, the liquid storage tube includes an inner sleeve and an outer sleeve sleeved around the inner sleeve. The liquid storage chamber is formed by a space between the outer sleeve and the inner sleeve. The liquid outlet device is received in the liquid storage chamber and sleeved around the inner sleeve. Both the elastic member and the first magnetic member are provided in a space between the inner sleeve and the liquid outlet device.

Further, the liquid outlet hole is defined in the inner sleeve. The liquid guiding hole is defined in a sidewall of the liquid outlet device corresponding to the liquid outlet hole.

An atomizer includes the foregoing cigarette cartridge. The atomizer further includes an atomizing assembly connected to the cigarette cartridge. The atomizing assembly includes a second magnetic member. The second magnetic member and the first magnetic member are mutually repulsive. When the atomizing assembly is assembled to the cigarette cartridge, a repulsive force between the second magnetic member and the first magnetic member is greater than an elastic force of the elastic member, so that the first magnetic member moves and compresses the elastic member to open the liquid guiding hole, causing the liquid storage chamber to be in communication with the liquid outlet hole.

Further, the atomizing assembly includes an atomizing end cover connected to the cigarette cartridge. An interior space of the atomizing end cover forms an atomizing chamber. The atomizing end cover defines, corresponding to the liquid outlet hole, a liquid intake hole in communication with the atomizing chamber. The second magnetic member is fixedly received in the atomizing chamber.

Further, the atomizing assembly includes an atomizing end cover connected to the cigarette cartridge. An interior space of the atomizing end cover forms an atomizing chamber. The atomizing end cover defines, corresponding to the liquid outlet hole, a liquid intake hole in communication with the atomizing chamber. The second magnetic member is provided outside the atomizing end cover and fixedly provided on the upper end of the atomizing end cover.

Further, the atomizing assembly includes a connecting seat and an atomizing head. The connecting seat is connected to the cigarette cartridge. The atomizing head is fixedly provided in the inner sleeve. An atomizing chamber is defined in the atomizing head. The atomizing head defines, corresponding to the liquid outlet hole, a permeable hole in communication with the atomizing chamber. The second magnetic member is fixedly received in the connecting seat.

An electronic cigarette includes the foregoing atomizer.

The advantages of the present application are described as follows. In the cigarette cartridge provided by the present application, when the cigarette cartridge is placed separately, because the first magnetic member is subject to an elastic action of the elastic member to seal the liquid guiding hole, thus the liquid storage chamber is isolated from the liquid outlet hole. A leakage of the cigarette liquid and an accidental ingestion of the cigarette liquid can be avoided. It plays a certain role in protecting children.

### BRIEF DESCRIPTION OF THE DRAWINGS

Exemplary embodiments of the present application are described more fully hereinafter with reference to the accompanying drawings.
FIG. 1 is a schematic view of an electronic cigarette according to a first embodiment of the present application;
FIG. 2 is a schematic view of a cigarette cartridge of the electronic cigarette of FIG. 1;
FIG. 3 is a schematic view of an atomizing assembly and a battery assembly of the electronic cigarette of FIG. 1;
FIG. 4 is a schematic view of an electronic cigarette according to a second embodiment of the present application;
FIG. 5 is a schematic view of a cigarette cartridge of the electronic cigarette of FIG. 4;
FIG. 6 is a schematic view of an atomizing assembly and a battery assembly of the electronic cigarette of FIG. 4;
FIG. 7 is a schematic view of an electronic cigarette according to a third embodiment of the present application; and
FIG. 8 is a schematic view of a cigarette cartridge and an atomizing head of the electronic cigarette of FIG. 7.

The following table lists the various components and reference numerals in the figures.

| | | |
|---|---|---|
| electronic cigarette 100, 200, 300 | cigarette cartridge 101 | atomizing assembly 102 |
| atomizer 10 | housing 11 | liquid storage tube 12 |
| liquid outlet device 13 | elastic member 14 | first magnetic member 15 |
| connecting seat 21 | atomizing end cover 22 | heating structure 23 |
| second magnetic member 24 | battery housing 31 | battery 32 |
| battery switch 33 | mouthpiece connector 111 | liquid storage chamber 121 |
| air exhaust passage 122 | through hole 123 | liquid guiding hole 131 |
| first air intake hole 211 | second air intake hole 212 | smoke outlet hole 213 |
| atomizing chamber 221 | liquid intake hole 222 | battery assembly 30 |
| liquid outlet hole 132 | air exhaust tube 16 | atomizing head 25 |
| inner sleeve 124 | outer sleeve 125 | |

### DETAILED DESCRIPTION OF THE PREFERRED EMBODIMENTS

The present application is specifically illustrated with reference to accompanying drawings. The drawings are simplified schematic views which show fundamental structures of exemplary embodiments of the present application. Thus, merely the constructions related to the present application are shown.

### First embodiment

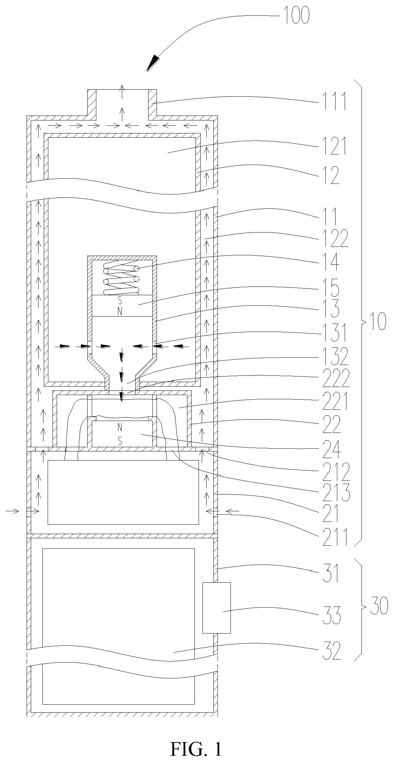

Referring to FIG. 1 through FIG. 3, an electronic cigarette 100 is provided by the first embodiment of the present application. The electronic cigarette 100 includes an atomizer 10 and a battery assembly 30 connected to the atomizer 10. The atomizer 10 includes a cigarette cartridge 101 and an atomizing assembly 102 detachably provided at a lower end of the cigarette cartridge 101. The battery assembly 30 is fixedly provided at a lower end of the atomizing assembly 102. The atomizing assembly 102 is capable of absorbing the cigarette liquid in the cigarette cartridge 101, and under the electric driving of the battery assembly 30, the cigarette liquid is heated and atomized to form smoke to be inhaled by a user.

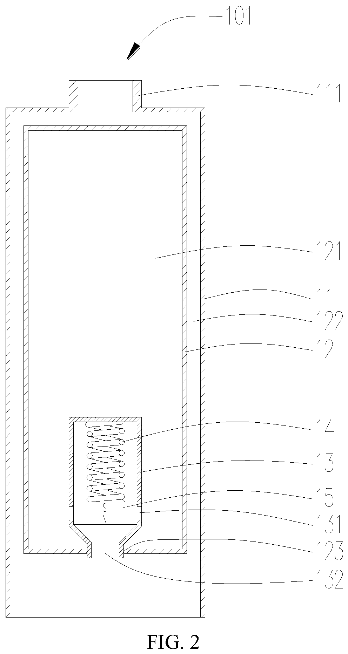

Referring to FIG. 2, specifically, the cigarette cartridge 101 includes a housing 11, a liquid storage tube 12 fixedly received in the housing 11, a liquid outlet device 13, an elastic member 14, and a first magnetic member 15. The liquid outlet device 13, the elastic member 14 and the first magnetic member 15 are received in the liquid storage tube 12. The elastic member 14 is received in the liquid outlet device 13. The first magnetic member 15 is slidably received in the liquid outlet device 13.

The housing 11 substantially has a hollow cylindrical structure. The housing 11 has an opening at a lower end. The housing 11 forms a mouthpiece connector 111 extending upward from the upper end of the housing 11 and along an axial direction of the housing 11. The mouthpiece connector 111 is in communication with the interior chamber of the housing 11. In the illustrated embodiment, when the user inhales, the mouthpiece connector 111 is held in the user's mouth directly for inhaling the smoke. It can be understood that, in alternative embodiments not shown, connecting members (not shown) can also be connected to the mouthpiece connector 111, and the user holds the connecting member in mouth to perform an inhaling of the smoke.

The liquid storage tube 12 substantially has a hollow tubular structure. The interior space of the liquid storage tube 12 forms a liquid storage chamber 121 for storing cigarette liquid. An air exhaust passage 122 is formed between the liquid storage tube 12 and the housing 11, wherein the air exhaust passage 122 is in communication with the mouthpiece connector 111.

It can be understood that, the liquid storage tube 12 is fixedly received in the housing 11 by an interference fit. The external wall of the liquid storage tube 12 defines at least one groove (not shown) along an axial direction of the liquid storage tube 12. At this time, the air exhaust passage 122 is formed by the space between the groove and the internal wall of the housing 11. Or, at least one fixing member such as a connecting rod (not shown) is provided between the external wall of the liquid storage tube 12 and the internal wall of the housing 11, one end of the fixing member is fixedly connected to the liquid storage tube 12 and an opposite end of the fixing member is fixedly connected to the housing 11, thereby allowing the liquid storage tube 12 to be fixedly received in the housing 11. At this time, the air exhaust passage 122 is formed by the space between the external wall of the liquid storage tube 12 and the internal wall of the housing 11.

Both the housing 11 and the liquid storage tube 12 are made of transparent material or translucent material, for facilitating the user to observe the residual amount of the cigarette liquid. In the illustrated embodiment, both the housing 11 and the liquid storage tube 12 are made of glass material.

Further, the lower end of the liquid storage tube 12 defines a though hole 123 in communication with the liquid storage chamber 121.

The liquid outlet device 13 is received in the liquid storage chamber 121. The liquid outlet device 13 substantially has a hollow bottle-like structure. The lower end of the liquid outlet device 13 defines a liquid outlet hole 132 in communication with the interior space of the liquid outlet device 13. The lower end of the liquid outlet device 13 fixedly extends into the through hole 123 of the liquid storage tube 12. The sidewall of the liquid outlet device 13 defines at least one liquid guiding hole 131, the liquid guiding hole 131 is in communication with the liquid storage chamber 121 and the interior space of the liquid outlet device 13 simultaneously. In the illustrated embodiment, the number of the liquid guiding hole 131 is two, the two liquid guiding holes 131 are provided oppositely on the liquid outlet device 13. It can be understood that, in alternative embodiments not shown, the number of the liquid guiding hole 131 can be one, three or more than three.

The elastic member 14 is retractable along the axial direction of the liquid storage tube 12. One end of the elastic member 14 elastically resists a top of the liquid outlet device 13, the other end of the elastic member 14 elastically resists the first magnetic member 15. The external wall of the first magnetic member 15 tightly contacts the internal wall of the liquid outlet device 13, so as to be able to close the liquid guiding hole 131 of the liquid outlet device 13. When the cigarette cartridge 101 is disassembled and placed separately, due to the elastic force of the elastic member 14, the first magnetic member 15 is moved downwardly, until the first magnetic member 15 closes the liquid guiding hole 131. At this time, the liquid storage chamber 121 is closed, the cigarette liquid in the liquid storage chamber 121 cannot flow into the liquid outlet device 13 via the liquid guiding hole 131.

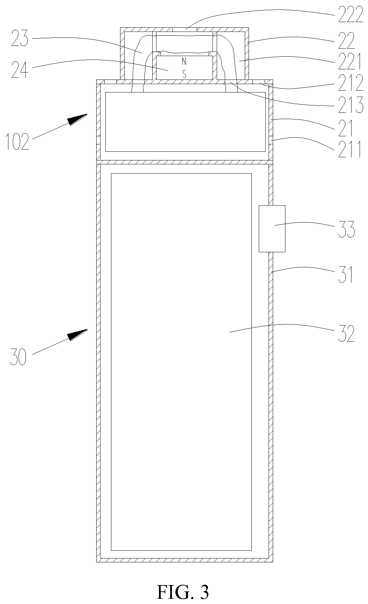

Referring to FIG. 3, specifically, the atomizing assembly 102 includes a connecting seat 21 detachably connected to the lower end of the housing 11, an atomizing end cover 22 provided at an upper end of the connecting seat 21, a heating structure 23 and a second magnetic member 24. The heating structure 23 and the second magnetic member 24 are received in the atomizing end cover 22.

The connecting seat 21 substantially has a cylindrical structure. The upper end of the connecting seat 21 is detachably connected to the lower end of the housing 11. It can be understood that, by providing threads, the connecting seat 21 can be connected to the housing 11 by a threaded connection. Or, by providing a lathing groove and a latching tab, a latching connection can be established between the connecting seat 21 and the housing 11. Or, connecting portions between the connecting seat 21 and the housing 11 can be provided with magnetic materials, and a magnetic connection can be established between the connecting seat 21 and the housing 11. The detachable connection structures between the connecting seat 21 and the housing 11 are not limited herein.

Further, the sidewall of the connecting seat 21 defines a first air intake hole 211. The upper end surface of the connecting seat 21 defines at least one second air intake hole 212 in communication with the first air intake hole 211 and the air exhaust passage 122 simultaneously.

The atomizing end cover 22 substantially has a cylindrical structure. The interior space of the atomizing end cover 22 forms an atomizing chamber 221. Further, in order for delivering the smoke generated in the atomizing end cover 22 into the user's mouth, the upper end surface of the connecting seat 21 defines at least one smoke outlet hole 213 corresponding to the atomizing chamber 221, and the smoke outlet hole 213 is in communication with the atomizing chamber 221. It can be understood that, in alternative embodiments not shown, the smoke outlet hole 213 can also be provided on the sidewall of the atomizing end cover 22, and the smoke outlet hole 213 is in communication with the air exhaust passage 122, the target of delivering the smoke into the user's mouth can also be realized.

Further, the upper end surface of the atomizing end cover 22 defines a liquid intake hole 222 corresponding to the liquid outlet hole 132 of the liquid outlet device 13, and the liquid intake hole 222 is in communication with the atomizing chamber 221.

The heating structure 23 is fixedly provided in the atomizing chamber 221. The heating structure 23 includes a heating member (not labeled) and a liquid guiding member (not labeled) which are wrapped around each other. The liquid guiding member is arranged corresponding to the liquid intake hole 222 for absorbing the cigarette liquid. The heating member is electrically connected to the battery assembly 30. It can be understood that, the liquid guiding member can be a member having a capacity of absorbing the cigarette liquid, such as a cotton or a porous ceramic and so on. The heating member can be a member such as a heating wire or a heating plate.

The second magnetic member 24 is fixedly provided in the atomizing chamber 221, and the second magnetic member 24 and the first magnetic member 15 are mutually repulsive. Further, when the cigarette cartridge 101 is assembled to the atomizing assembly 102, the repulsive force between the second magnetic member 24 and the first magnetic member 15 is greater than the elastic force of the elastic member 14, thereby driving the first magnetic member 15 to move upward.

In the illustrated embodiment, both the first magnetic member 15 and the second magnetic member 24 are bar-type magnets. The north poles N of the first magnetic member 15 and the second magnetic member 24 face each other, to establish a repulsive force. It can be understood that, the shape of the magnets can be cylindrical shape or other shape and is not limited herein, so long as the first magnetic member 15 and the second magnetic member 24 repel each other. It can also be understood that, in alternative embodiments not shown, the south poles S of the first magnetic member 15 and the second magnetic member 24 face each other. It can also be understood that, the first magnetic member 15 and the second magnetic member 24 can also be electromagnets, a repulsive action between the first magnetic member 15 and the second magnetic member 24 is established by a control of the spiral orientation of the coil or the current flowing through the coil.

The battery assembly 30 includes a battery housing 31, a battery 32 and a battery switch 33. The upper end of the battery housing 31 is fixedly connected to the lower end of the atomizing end cover 22. The battery 32 is fixedly received in the battery housing 31, and the battery 32 is electrically connected to the heating member. The battery switch 33 is provided on the battery housing 31, and the battery switch 33 is electrically connected to the battery 32, for controlling the power supplying of the battery 32 for the heating member.

The assembly and the usage of the electronic cigarette 100 of the present application is illustrated with the accompanying drawings as follows.

In the electronic cigarette 100 provided by the present application, when it leaves factory, the atomizing assembly 102 has already been fixedly connected to the battery assembly 30, as shown in FIG. 3. When the user wants to inhale, the cigarette cartridge 101 is merely required to be aligned with and assembled to the atomizing assembly 102.

Referring to FIG. 2, when the cigarette cartridge 101 is placed separately, due to an elastic force of the elastic member 14, the first magnetic member 15 is moved downward under the elastic action. When the first magnetic member 15 moves to resist the lower end of the liquid outlet device 13, the liquid guiding hole 131 of the liquid outlet device 13 is closed by the first magnetic member 15, thereby closing the liquid storage chamber 121, the liquid storage chamber 121 is isolated from the liquid outlet hole 132, and the cigarette liquid cannot flow out.

Referring to FIG. 1, when the cigarette cartridge 101 is aligned with and assembled to the atomizing assembly 102, the atomizing assembly 102 approaches towards the cigarette cartridge 101 gradually, causing the second magnetic member 24 to approach the first magnetic member 15 gradually. When the repulsive force between the second magnetic member 24 and the first magnetic member 15 is greater than the elastic force of the first elastic member 14, the first magnetic member 15 is enabled to move upward. At this time, the liquid guiding hole 131 of the liquid outlet device 13 is opened gradually, the liquid storage chamber 121 is opened to communicate with the liquid outlet device 13. The cigarette liquid flows into the liquid outlet device 13 via the liquid guiding hole 131, and then flows out from the liquid outlet hole 132 and is absorbed by the liquid guiding member by passing through the liquid intake hole 222. The cigarette liquid is heated and atomized to form smoke under the electric driving of the heating member, the smoke fills up the atomizing chamber 221. The direction indicated by the thicker arrows in FIG. 1 is the flowing direction of the cigarette liquid.

When the user inhales, external air flows into the connecting seat 21 via the first air intake hole 211, the smoke in the atomizing chamber 221 also flows into the connecting seat 21 via the smoke outlet hole 213, the air mixed with the smoke then enters into the user's mouth by passing through the second air intake hole 212, the air exhaust passage 122 and the mouthpiece connector 111 successively. The direction indicated by the thinner arrows in FIG. 1 is the flowing direction of the air.

When the cigarette cartridge 101 is disassembled and separated from the atomizing assembly 102, the second magnetic member 24 moves away from the first magnetic member 15 gradually, the repulsive force between the second magnetic member 24 and the first magnetic member 15 decreases gradually until it goes down to zero. At this time, due to an elastic force of the elastic member 14, the first magnetic member 15 is moved downward until the first magnetic member 15 closes the liquid guiding hole 131 again. The liquid storage chamber 121 is isolated from the liquid guiding hole 131 again, and the cigarette liquid cannot flow out. At this time, the cigarette cartridge 101 is in the state shown in FIG. 2. Therefore, even if the children open the cigarette cartridge 101 casually, a leakage of the cigarette liquid cannot occur, an accidental ingestion of the cigarette liquid by the children is avoided, thereby playing a certain role in protecting children. Further, the liquid storage chamber 121 is closed by the first magnetic member 15, compared to the traditional structure, the first magnetic member 15 does not deteriorate, and a service life is prolonged.

It can be understood that, in alternative embodiments not shown, a part of the liquid guiding member can further extend out of the atomizing end cover 22 by passing through the liquid intake hole 222. When the cigarette cartridge 101 is connected to the atomizing assembly 102, a part of the liquid guiding member extends into the liquid outlet device 13, allowing the cigarette liquid to be absorbed by the liquid guiding member gradually, to prevent excessive cigarette liquid from flowing into the atomizing assembly 102.

In the atomizer 10 provided by the first embodiment of the present application, according to the homogeneous repulsion principle of the magnetic poles, the movement of the first magnetic member 15 is controlled, thereby the liquid storage chamber 121 can be opened when the cigarette cartridge 101 is assembled, and the liquid storage chamber 121 can be closed when the cigarette cartridge 101 is disassembled. When the cigarette cartridge 101 is placed separately, a leakage of the cigarette liquid or an accidental ingestion of the cigarette liquid by the children can be avoided.

In the electronic cigarette 100 provided by the first embodiment of the present application, because the electronic cigarette 100 includes all the technical features of aforementioned atomizer 10, thus the electronic cigarette 100 has all the technical effects same as those of aforementioned atomizer 10.

### Second embodiment

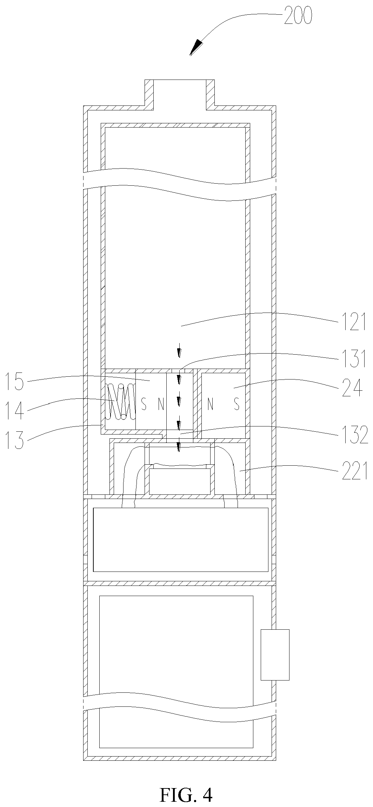

Referring to FIG. 4 through FIG. 6, the main difference between the electronic cigarette 200 provided by the second embodiment and the electronic cigarette 100 provided by the first embodiment is that, the liquid outlet device 13 is provided outside the liquid storage tube 12, and the second magnetic member 24 is provided outside the atomizing end cover 22.

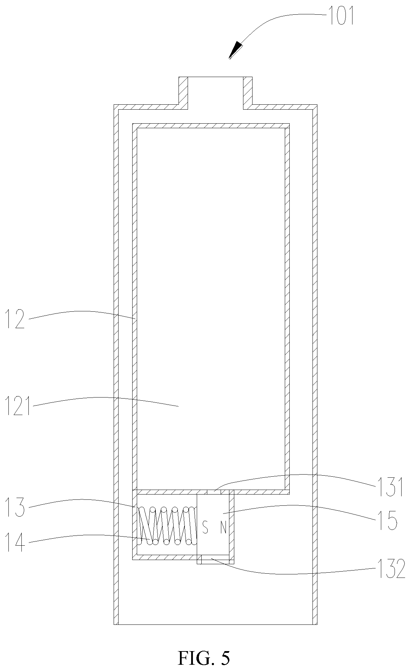

Specifically, referring to FIG. 5, the liquid outlet device 13 is fixedly connected to the lower end surface of the liquid storage tube 12. The liquid guiding hole 131 is defined in the connecting surface between the liquid outlet device 13 and the liquid storage tube 12. The liquid outlet hole 132 is defined in the liquid outlet device 13 corresponding to the liquid guiding hole 131, and the liquid outlet hole 132 is in communication with the liquid guiding hole 131. The elastic member 14 is retractable along the radial direction of the liquid storage tube 12. One end of the elastic member 14 elastically resists the liquid outlet device 13, and the other end of the elastic member 14 elastically resists the first magnetic member 15. The first magnetic member 15 is slidably received in the liquid outlet device 13, and is slidable along a direction perpendicular to the axial direction of the liquid storage tube 12. When the cigarette cartridge 101 is placed separately, due to an elastic action of the elastic member 14, the first magnetic member 15 is fixedly resisted, the first magnetic member 15 seals the liquid guiding hole 131 and the liquid outlet hole 132, the liquid storage chamber 121 is isolated from the liquid outlet hole 132, so that the cigarette liquid cannot flow out.

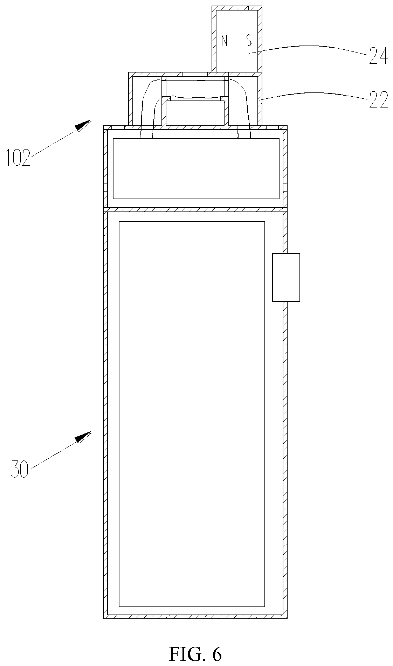

Referring to FIG. 6, the second magnetic member 24 is fixedly provided on the upper end surface of the atomizing end cover 22. Referring to FIG. 4, in normal use, the cigarette cartridge 101 is connected to the atomizing assembly 102, the second magnetic member 24 is located on a side of the first magnetic member 15 opposite to the elastic member 14. The second magnetic member 24 and the first magnetic member 15 are mutually repulsive. The repulsive force between the second magnetic member 24 and the first magnetic member 15 is greater than the elastic force of the first elastic member 14, thus the first magnetic member 15 moves and compresses the elastic member 14, and the liquid guiding hole 131 is opened finally, causing the liquid outlet hole 132 to be in communication with the liquid storage chamber 121. At this time, the cigarette liquid in the liquid storage chamber 121 flows into the liquid outlet device 13 via the liquid guiding hole 131, and then the cigarette liquid flows into the atomizing chamber 221 via the liquid outlet hole 132. The direction by the arrows in FIG. 4 indicates the flowing direction of the cigarette liquid.

In the illustrated embodiment, the north poles N of the first magnetic member 15 and the second magnetic member 24 face each other. It can be understood that, in alternative embodiments not shown, the south poles S of the first magnetic member 15 and the second magnetic member 24 face each other. It can also be understood that, the first magnetic member 15 and the second magnetic member 24 can also be electromagnets, a repulsive action between the first magnetic member 15 and the second magnetic member 24 is established by a control of the spiral orientation of the coil or the current flowing through the coil.

Referring to FIG. 5 again, when the cigarette cartridge 101 is separated from the atomizing assembly 102, the first magnetic member 15 is moved back to an original position due to an elastic action of the elastic member 14, finally enabling the first magnetic member 15 to seal the liquid guiding hole 131, and the liquid storage chamber 121 is isolated from the liquid outlet hole 132 again. When the cigarette cartridge 101 is placed separately, a leakage of the cigarette liquid and an accidental ingestion of the cigarette liquid by the children can be avoided.

The structure of the battery assembly 30 of the electronic cigarette 200 provided by the second embodiment is the same as that of the battery assembly 30 in the first embodiment, which is not specifically described again.

### Third embodiment

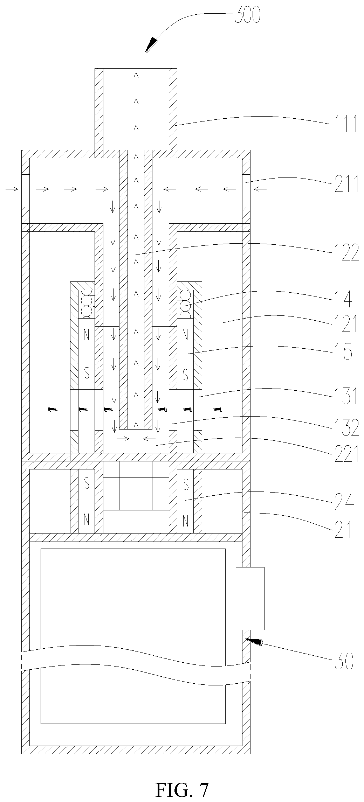

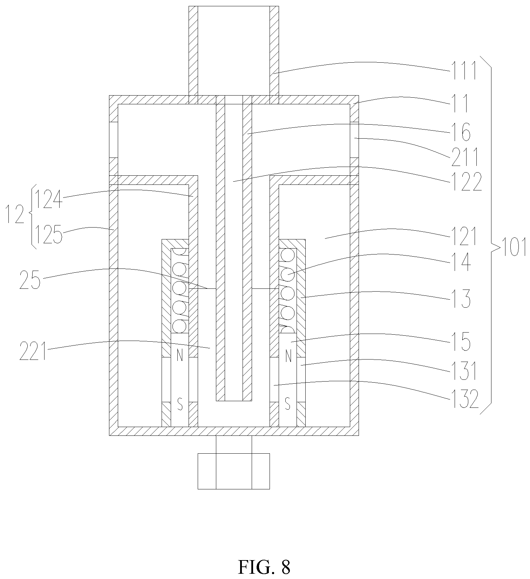

Referring to FIG. 7 and FIG. 8, the main difference between the electronic cigarette 300 provided by the third embodiment and the electronic cigarette 100 provided by the first embodiment is that: the air intake manner and the liquid intake manner are different. In the first embodiment, the air enters from the lower end, and the liquid enters from the upper end, while in the third embodiment, the air enters from the upper end, and the liquid enters from the sidewall.

Specifically, referring to FIG. 8, the cigarette cartridge 101 includes a housing 11, a liquid storage tube 12 provided in the housing 11, a liquid outlet device 13 received in the liquid storage tube 12, an elastic member 14, a first magnetic member 15, and an air exhaust tube 16 connected to the housing 11. The elastic member 14 and the first magnetic member 15 are received in the liquid outlet device 13.

The liquid storage tube 12 substantially has a columnar structure. The liquid storage tube 12 includes an inner sleeve 124 and an outer sleeve 125 sleeved around the inner sleeve 124. A space between the inner sleeve 124 and the outer sleeve 125 forms a liquid storage chamber 121. The liquid outlet device 13 is fixedly received in the liquid storage chamber 121, and the liquid outlet device 13 is sleeved around the inner sleeve 124. The liquid guiding hole 131 is defined in the sidewall of the liquid outlet device 13, the liquid outlet hole 132 is defined in the inner sleeve 124 corresponding to the liquid guiding hole 131, and the liquid guiding hole 131 is in communication with the liquid outlet hole 132. The elastic member 14 and the first magnetic member 15 are received in a space formed between the inner sleeve 124 and the liquid outlet device 13. One end of the elastic member 14 elastically resists the liquid outlet device 13, and the other end of the elastic member 14 elastically resists the first magnetic member 15. The first magnetic member 15 is slidably received in the liquid outlet device 13. When the cigarette cartridge 101 is placed separately, due to an elastic action of the elastic member 14, the first magnetic member 15 is fixedly resisted, the first magnetic member 15 seals the liquid guiding hole 131. At this time, the liquid storage chamber 121 is closed, and the liquid storage chamber 121 is isolated from the liquid outlet hole 132, thus the cigarette liquid in the liquid storage chamber 121 cannot flow into the liquid outlet device 13 via the liquid guiding hole 131.

Referring to FIG. 7 and FIG. 8, the atomizing assembly 102 of the illustrated embodiment includes a connecting seat 21, an atomizing head 25, and a second magnetic member 24.

Referring to FIG. 8, the atomizing head 25 is fixedly provided in the inner sleeve 124. The atomizing head 25 defines an atomizing chamber 221 therein, and the atomizing head 25 defines a permeable hole (not shown) corresponding to the liquid outlet hole 132 and in communication with the atomizing chamber 221. Referring to FIG. 7, the second magnetic member 24 is fixedly provided in the connecting seat 21. When the connecting seat 21 is assembled to the cigarette cartridge 101, because the second magnetic member 24 and the first magnetic member 15 are mutually repulsive, and the repulsive force between the second magnetic member 24 and the first magnetic member 15 is greater than the elastic force of the first elastic member 14, thus the first magnetic member 15 moves and compresses the elastic member 14, the first magnetic member 15 finally opens the liquid guiding hole 131, thereby causing the liquid storage chamber 121 to be in communication with the liquid outlet hole 132. At this time, the cigarette liquid in the liquid storage chamber 121 flows into the liquid outlet device 13 via the liquid guiding hole 131, and then flows into the atomizing head 25 by passing through the liquid outlet hole 132 and the permeable hole successively, so that the cigarette liquid is heated and atomized by the heating structure (not shown) in the atomizing head 25 to form smoke. The direction indicated by the thicker arrows in FIG. 7 is the flowing direction of the cigarette liquid.

In the illustrated embodiment, the south poles S of the first magnetic member 15 and the second magnetic member 24 face each other. It can be understood that, in alternative embodiments not shown, the north poles N of the first magnetic member 15 and the second magnetic member 24 face each other. It can also be understood that, the first magnetic member 15 and the second magnetic member 24 can also be electromagnets, a repulsive action between the first magnetic member 15 and the second magnetic member 24 is established by a control of the spiral orientation of the coil or the current flowing through the coil.

Referring to FIG. 8, the housing 11 has a structure the same as that in the first embodiment, which is not specifically described herein. One end of the air exhaust tube 16 is connected to the housing 11 corresponding to the mouthpiece connector 111, and the other end of the air exhaust tube 16 is inserted into the atomizing head 25. The interior space of the air exhaust tube 16 forms an air exhaust passage 122, the air exhaust passage 122 is in communication with the mouthpiece connector 111 and the atomizing chamber 221 simultaneously.

Referring to FIG. 7, the internal wall of the housing 11 tightly contacts the external annular surface of the liquid storage tube 12. The external wall of the housing 11 defines a first air intake hole 211 above the liquid storage tube 12 and in communication with the atomizing chamber 221. When the user inhales, the external air flows into the atomizing chamber 221 via the first air intake hole 211, the air is mixed with the smoke and then flows into the user's mouth by passing through the air exhaust passage 122 and the mouthpiece connector 111 successively. The direction indicated by the thinner arrows in FIG. 7 is the flow direction of the air.

It can be understood that, a part of the inner sleeve 124 clamped between the atomizing head 25 and the liquid outlet device 13 can be omitted. At this time, a side of the first magnetic member 15 contacts the internal wall of the liquid outlet device 13 to close the liquid guiding hole 131, and another side of the first magnetic member 15 away from the liquid outlet device 13 contacts the sidewall of the atomizing head 25 to close the permeable hole. When the cigarette cartridge 101 is connected to the atomizing assembly 102, the first magnetic member 15 is moved upward to open the liquid guiding hole 131 and the permeable hole. The cigarette liquid flows into the interior of the atomizing head 25 by passing through the liquid guiding hole 131 and the permeable hole, and is atomized to form smoke.

In the battery assembly 30 of the electronic cigarette 300 of the third embodiment, the first air intake hole 211 in the battery housing 31 is omitted, the other structures of the battery assembly 30 are the same as those in the first embodiment, which is not specifically described herein.

The embodiments described above are merely preferred embodiments, but not intended to limit the application. Any modifications, alternatives or improvements made within the principle of the present application should be interpreted as falling within the protection scope of the present application. The claims are not limited to the features or acts described above. Rather, the proper scope of the present application is defined by the appended claims.

## Claims

1. A cigarette cartridge comprising a liquid storage tube, a liquid outlet device, an elastic member and a first magnetic member, wherein the elastic member and the first magnetic member are received in the liquid outlet device, a liquid storage chamber is provided in the liquid storage tube, the liquid outlet device is provided with a liquid guiding hole in communication with the liquid storage chamber, the cigarette cartridge is further provided with a liquid outlet hole in communication with the liquid guiding hole, the first magnetic member is slidably received in the liquid outlet device, one end of the elastic member elastically resists an inner wall of the liquid outlet device, the other end of the elastic member elastically resists the first magnetic member, the first magnetic member is capable of closing the liquid guiding hole under an action of the elastic member.

2. The cigarette cartridge according to claim **1**, wherein the liquid outlet device is received in the liquid storage tube, the first magnetic member is slidable along an axial direction of the liquid storage tube.

3. The cigarette cartridge according to claim **2**, wherein the liquid guiding hole is defined in a sidewall of the liquid outlet device, the liquid outlet hole is defined at a lower end of the liquid outlet device.

4. The cigarette cartridge according to claim **2** or **3**, wherein a lower end of the liquid storage tube defines a though hole in communication with the liquid storage chamber, a lower end of the liquid outlet device fixedly extends into the through hole of the liquid storage tube.

5. The cigarette cartridge according to claim **1**, wherein the liquid outlet device is provided outside the liquid storage tube and connected to a lower end of the liquid storage tube, the first magnetic member is slidable along a radial direction of the liquid storage tube.

6. The cigarette cartridge according to claim **5**, wherein the liquid guiding hole is defined in a connecting surface between the liquid outlet device and the liquid storage tube, the liquid outlet hole is defined in the liquid outlet device corresponding to the liquid guiding hole.

7. The cigarette cartridge according to claim **1**, wherein the liquid storage tube comprises an inner sleeve and an outer sleeve sleeved around the inner sleeve, the liquid storage chamber is formed by a space between the outer sleeve and the inner sleeve, the liquid outlet device is received in the liquid storage chamber and sleeved around the inner sleeve, both the elastic member and the first magnetic member are provided in a space between the inner sleeve and the liquid outlet device.

8. The cigarette cartridge according to claim **7**, wherein the liquid outlet hole is defined in the inner sleeve, the liquid guiding hole is defined in a sidewall of the liquid outlet device corresponding to the liquid outlet hole.

9. An atomizer comprising the cigarette cartridge according to any one of claims **1** to **8**, wherein the atomizer further comprises an atomizing assembly connected to the cigarette cartridge, the atomizing assembly comprises a second magnetic member, the second magnetic member and the first magnetic member are mutually repulsive, when the atomizing assembly is assembled to the cigarette cartridge, a repulsive force between the second magnetic member and the first magnetic member is greater than an elastic force of the elastic member, so that the first magnetic member moves and compresses the elastic member to open the liquid guiding hole, causing the liquid storage chamber to be in communication with the liquid outlet hole.

10. The atomizer according to claim **9**, wherein the atomizing assembly comprises an atomizing end cover connected to the cigarette cartridge, an interior space of the atomizing end cover forms an atomizing chamber, the atomizing end cover defines, corresponding to the liquid outlet hole, a liquid intake hole in communication with the atomizing chamber, the second magnetic member is fixedly received in the atomizing chamber.

11. The atomizer according to claim **9**, wherein the atomizing assembly comprises an atomizing end cover connected to the cigarette cartridge, an interior space of the atomizing end cover forms an atomizing chamber, the atomizing end cover defines, corresponding to the liquid outlet hole, a liquid intake hole in communication with the atomizing chamber, the second magnetic member is provided outside the atomizing end cover and fixedly provided on the upper end of the atomizing end cover.

12. The atomizer according to claim **9**, wherein the atomizing assembly comprises a connecting seat and an atomizing head, the connecting seat is connected to the cigarette cartridge, the atomizing head is fixedly provided in the inner sleeve, an atomizing chamber is defined in the atomizing head, the atomizing head defines, corresponding to the liquid outlet hole, a permeable hole in communication with the atomizing chamber, the second magnetic member is fixedly received in the connecting seat.

13. An electronic cigarette comprising the atomizer according to any one of claims **9** to **12**.
